(19) Europäisches Patentamt
European Patent Office
Office européen des brevets

(11) Veröffentlichungsnummer : **0 439 640 B1**

(12) **EUROPÄISCHE PATENTSCHRIFT**

(45) Veröffentlichungstag der Patentschrift :
**15.09.93 Patentblatt 93/37**

(51) Int. Cl.⁵ : **A61K 33/06,** A61K 33/32,
// (A61K33/32, 33:24, 33:06)

(21) Anmeldenummer : **90101706.1**

(22) Anmeldetag : **29.01.90**

(54) **Verfahren zur Herstellung von Mitteln zur Therapie von Hauterkrankungen.**

(43) Veröffentlichungstag der Anmeldung :
**07.08.91 Patentblatt 91/32**

(45) Bekanntmachung des Hinweises auf die
Patenterteilung :
**15.09.93 Patentblatt 93/37**

(84) Benannte Vertragsstaaten :
**AT BE CH DE FR IT LI LU NL**

(56) Entgegenhaltungen :
**EP-A- 0 312 697
EP-A- 0 363 696
DE-A- 1 906 155
FR-A- 2 122 613
FR-A- 2 612 775
DATABASE WPI/L, DERWENT, Nr. 87-134376,
Derwent Publications Ltd, London, GB**

(73) Patentinhaber : **WOGEPHARM GmbH
Afrastrasse 21
D-50354 Hürth (DE)**

(72) Erfinder : **Schneider, Karl
Ostlandstrasse 12
D-6427 Bad Salzschlirf (DE)**
Erfinder : **Diezel, Prof. Dr. sc. med.
Anton-Saefkow-Platz 3
O-1156 Berlin (DE)**
Erfinder : **Schröpl, Prof. Dr.
Nelkenstrasse 2
D-6201 Wiesbaden-Naurod (DE)**

(74) Vertreter : **Werner, Hans-Karsten et al
Patentanwälte von Kreisler-Selting-Werner,
Postfach 10 22 41, Bahnhofsvorplatz 1
D-50462 Köln (DE)**

EP 0 439 640 B1

**Beschreibung**

Die Erfindung betrifft ein Verfahren zur Herstellung von Mitteln zur Therapie bzw. Abschwächung allergischer, entzündlicher und autoimmunologischer Hauterkrankungen. Die Anwendungsgebiete der Erfindung erstrecken sich auf das Gesundheitswesen, die pharmazeutische und die kosmetische Industrie.

Bestimmte entzündliche Erkrankungen der menschlichen Haut, wie beispielsweise Ekzeme, möglicherweise auch die Psoriasis entstehen, wenn epidermale Langerhans-Zellen, im folgenden mit ELZ bezeichnet, Substanzen wie Fremdantigene oder körpereigene Moleküle, phagozytieren und sich als Folge zytotoxische T-Lymphozyten bilden, die gegen die urspünglich phagozytierten Moleküle gerichtet sind. Befinden sich die Moleküle auf der Zelloberfläche, werden die Zellen zerstört und es resultiert eine Entzündung.

Siehe hierzu die folgende übersicht: W. Diesel und G. Stingl: Dermatol. Monatsschr. 171 (1985) 303).

Der entzündliche Vorgang wird durch sogenannte "Entzündungsenzyme", primär Lipoxygenasen unterhalten.

Eine Hemmung entzündlicher Prozesse bei Hauterkrankungen wird bisher durch folgende Therapeutika bzw. Therapieprinzipien erreicht:

1. Glukokortikoide [M. Furne u. S.I. Katz: J. Invest. Dermatolog. 92 (1989) 342; W. Aberer et al.: J. Immunol. 136 (1986) 1210]
2. UV-Licht [D.V. Belsito et al.: J. Exp. Med. 155 (1982) 291; W. Aberer et al.: J. Invest. Dermatol. 76 (1981) 202]
3. Lipoxygenase-Hemmer [Kl. Kragballe: Arch. Dermator. 119 (1983) 548]; und
4. Pix Lithantracis (Teerpräparate) und Dithranol, eine übersicht findet sich bei: S. Gruner et. al.: Dermatol. Monatsschr. 173 (1987) 436).

Die genannten entzündungshemmenden therapeutischen Mittel haben jedoch bei äußerlicher Anwendung auch die folgenden Nachteile, nämlich:

zu 1.    Glukokortikoide: Hautatrophie, Hautalterung;

zu 2.    UV-Licht: Hautalterung, Provokation der Entstehung von Haut-Tumoren;

zu 3.    Lipoxygenase-Hemmer: geringe Wirksamkeit bei äußerlicher Anwendung und toxische Nebenwirkungen, und

zu 4.    Pix Lithanthracis und Dithranol: Hautverfärbung, Lichtsensibilisierung sowie Geruchsbelästigung.

Aus der EP-A1 0 217 975 ist es bekannt, zur Behandlung der Schuppenflechte (Psoriasis) und anderer Hauterkrankungen definierte Salzmischungen auf die erkrankte Haut aufzutragen. Die Salzmischungen weisen dabei (in g/Kg Salzmischung, Rest zu 1.000 g ist Kristallwasser) die folgende Zusammensetzung auf, nämlich: Magnesium, Natrium, Kalzium, Kalium, Strontium, Eisen, Aluminium, Zink und Lithiuum. Die Zusammensetzung dieses Salzes entspricht etwa der Zusammensetzung des im Wasser des Toten Meeres gelösten Salzes.

Es konnte festgestellt werden, daß einige dieser Salze für die beabsichtigte Therapie vorteilhaft sind, während andere Salze bestimmte Funktionen der Haut hemmen, wie im folgenden näher ausgeführt wird.

So benötigen entzündungsauslösende Zellen der Haut (Langerhans-Zellen) intrazellulär Ca-Ionen, um funktionstüchtig zu sein, d.h. um eine Entzündung auszulosen. Das Einströmen des Ca in die Zelle erfolgt durch Calcium-Kanäle. Es konnte festgestellt werden, daß diese Kanäle durch Magnesium blockiert werden.

Eine Reihe von "Entzündungsenzymen", nämlich primär die Lipoxygenasen, aber auch Phospholipasen benötigen als Co-Enzym Ca, um sehr aktiv zu sein. Das Ca wird hierbei durch Mg verdrängt und es resultiert eine Hemmung der "Entzündungsenzyme".

Der Erfindung liegt somit die Aufgabe zugrunde, der medizinischen Praxis neue Mittel in die Hand zu geben, die sich für die Therapie entzündlicher Hauterkrankungen, insbesondere von Ekzemen und der Psoriasis vulgaris (Schuppenflechte) besonders gut eignen. Die Erfindung geht dabei von der Erkenntnis aus, daß es zur Lösung der gestellten Aufgabe erforderlich ist:

A) die Funktion epidermaler Langerhans-Zellen (ELZ) gezielt zu erniedrigen und gleichzeitig

B) die Aktivität der epidermalen Lipoxygenasen und der Phospholipase A 2 ("Entzündungsenzyme" der Haut) zu hemmen.

Die Lösung dieser Aufgabe erfolgt bei einem Verfahren zur Herstellung von Mitteln zur Therapie entzündlicher Hautkrankheiten bzw. zur Abschwächung allergischer entzündlicher Raktionen der Haut dadurch, daß Magnesiumchlorid verwendet wird.

In Weiterbildung der Erfindung werden in vorteilhafter Weise auch Magnesium-Ionen in Kombination mit Cd-, Co- und Mn-Ionen oder in Kombination mit einem dieser Ionen verwendet.

Die Konzentration des Magnesiums liegt in einem Bereich von 1,0 bis 30,0 Gewichtsprozente, wobei das Magnesium in Salbengrundlagen, Lösungen, Wässer, Grundlagen für Zahncremes oder Kosmetika eingearbeitet ist.

Bei entzündlichen Hautkrankheiten wird das entsprechend eingearbeitete Magnesiumsalz äußerlich angewendet.

Nach der Erfindung eignet sich MgCl$_2$, gegeben falls in Kombination mit Mn-, Cd- und Co. Salzen, vorteilhaft für die Herstellung von Dermatika, Heilwässer, Zahncremes, Körperwaschmittel oder Kosmetika, so daß sie in Salbengrundlagen oder entsprechenden Trägersubstanzen inkorporiert werden, und in Form von Badezusätzen, Kopf- und Körperwaschmitteln, Zahncremes und Kosmetika verwendet werden können.

Das Wesen der Erfindung wird anhand einiger Ausführungsbeispiele näher beschrieben.

Epidermale Langerhans-Zellen (ELZ) sind an der Entstehung entzündlicher Hauterkrankungen ursächlich beteiligt, wie bei Ekzemen aller Art, die Psoriasis u.a..

Dieses Wissen basiert auf folgenden Erkenntnissen. Die Zellen besitzen auf ihrer Oberfläche Ia (beim Menschen HLA-DR) - Antigene

[L. Klareskog et al.: Nature 268 (1977) 248;

G. Rowden et al.: Nature 268 (1977) 245],

sie phagozytieren Antigenmoleküle und sie sensibilisieren spezifische Lymphozyten

[L. Perry: Clin. Immunol. Immunophatol. 24 (1982)

204; I. Silberberg et al.: Cell. Immunol. 18 (1975)

453; G. Toews et al. : J. Immunol. 124 1989) 445].

Danach gibt es zwei grundsätzliche Möglichkeiten bzw. Therapiemöglichkeiten, um die Entstehung entzündlicher Hauterkrankungen durch ELZ zu verhindern:

(1) Verminderung der Anzahl der Zellen in der Haut bzw. Alteration bestimmter Oberflächenantigene der Zellen (HLA-DR usw.),

(2) gezielte Beeinträchtigung der Funktion der ELZ. Eine Abnahme der Anzahl funktionstüchtiger ELZ wird gegenwärtig durch folgende Therapieprinzipien erreicht:

UV-Licht, einschl. PUVA;

[W. Aberer et al.: J. Invest. Dermatol. 76 (1981) 202; P.Friedman: Br. J. Dermatol. 105 (1981) 219; G. Strauss et al.: Lancet II (1980) 556)],

Glukokortikoide,

[D. Belsito et al.: J. Exp. Med. 155 (1982) 291; M. Furne u. S.I. Katz] und

Pix Lithanthracis [S. Gruner et al.: Dermatol. Monatsschr.

173 (1987) 436)].

Wie kürzlich festgestellt wurde, werden Makrophagen, d.h. antigenpräsentierende Zellen wie auch die ELZ, funktionell durch folgende Ionen in ihrer Funktion gehemmt: Mg, Mn, Cd, Co

[R.W. Tsien et al.: Ann. Rev. Biophys. Biophys. Chem. 16 (1987) 265; H.J. Woehlck et al.: Cell. Biol. Intl. Repts. 10 (1986) 517].

Daher sollten die o.g. Ionen bzw. alle Salze dieser Ionen durch ELZ initiierte Hautentzündungen hemmen. Die Hemmung wird durch äußerliche Anwendung erreicht. Weiterhin wurde kürzlich bekannt, daß epidermale Lipoxygenasen und die Phospholipase A 2 als Co-Enzyme Ca-Ionen benötigen, um voll funktionstüchtig zu sein

[(B.A. Burrall et al.: J. Invest. Dermatol.91 (1988) 294].

Lipoxygenase- und die Phospholipase-Produkte sind an allen entzündlichen Vorgängen ursächlich beteiligt

[Übersicht: Ruzicka et al.: Rev. Physiol. Biochem. Pharmacol. 100 (1984) 122].

Als Folge der Verdrängung des Calciums vor der "prosthetischen" Gruppe der Lipoxygenasen resultiert demnach eine kompetitive Enzymhemmung und ein Abbruch aller durch Lipoxygenasen initiierten entzündlichen Prozesse. Eine Verdrängung des Calciums kann generell durch Magnesium-Ionen erreicht werden

[Übersicht: E. Carafoli u. J.T. Penniston: Spektrum der Wissenschaft, Januar 1986; S. 76-85].

Nach der Erfindung sind daher primär Mg und dessen Salze aufgrund der Hemmung der Funktion ELZ und der Hemmung der Aktivität von Lipoxygenasen nach äußerlicher Anwendung bei entzündlichen Hauterkrankungen therapiewirksam. Die äußerliche Anwendung erfolgt nach Inkorporation der o.g. Ionen in Salben, Badezusätze, Zahncremes, Kopf- bzw. Körperwaschmittel und Kosmetika.

Zur Beweisführung für die entzündungshemmende Wirkung von Magnesium-Ionen nach äußerlicher Anwendung wurden folgende tierexperimentelle Untersuchungen durchgeführt:

BALB/c-Mäuse wurden gegenüber 1-Chloro-2.4-Dinitrobenzen (DNCB) artifiziell immunisiert. Nach der Immunisierung wurden die Ohren der Tiere äußerlich mit DNCB behandelt. Als Folge resultiert eine entzündliche Reaktion und eine Zunahme der Ohrdicke. Diese ist ein Maß für die Intensität der entzündlichen Reaktion [G.H. Strauss et al.: Lancet II (1980) 556].

Wie aus der Tabelle 1 ersichtlich ist, hemmt MgCl$_2$ die durch DNCB initiierte entzündlichen Reaktionen entscheidend.

Weiterhin wurde nachgewiesen, daß MgCl$_2$ auch Hautentzündungen beim Menschen hemmt. Hierfür wur-

3

den Patienten mit einer Formaldehyd-bzw. Nickel-Allergie zur epikutanen Testung mit Formaldhyd bzw. Nickel in Kontakt gebracht, wodurch sich eine massive Hautentzündung ergab. Es ergab sich jedoch dann eine sehr abgeschwächte Entzündung, wenn den gleichen Patienten Formaldehyd bzw. Nickel in Kombination mit $MgCl_2$ verabreicht wurden. Im Gegensatz zum $MgCl_2$, hemmte NaCl die Entzündung nicht.

Ausführungsbeispiele

Beispiel 1:

Kontaktüberempfindlichkeit gegen 1-Chloro-2.4-Dinitrobenzen (DNCB) - Nachweis und Hemmung durch $MgCl_2$

BALB/c-Mäuse, und zwar 5 Tiere pro Tiergruppe, wurden folgendermaßen gegen DNCB sensibilisiert: Applikation von 25 µl einer 1%igen DNCB-Lösung (DNCB, gelöst in Aceton-Olivenöl, 4:1) an drei aufeinanderfolgenden Tagen (Tage-3, -2, -1) auf ein Hautareal des Rückens (0,5 cm x 0,5 cm) nach vorheriger Rasur. Nach fünf Tagen (Tag +5) wurde bei den gleichen Tieren 25 µl einer 0, 5%igen, 0,25%igen oder 0, 125%igen DNCB Lösung ohne bzw. mit einem unterschiedlichen $MgCl_2$-Gehalt (Tabelle 1) auf die dorsale Oberfläche beider Ohren aufgetragen (Prä-DNCB-Meßwert).

Die DNCB und $MgCl_2$ enthaltende Lösung wurde folgendermaßen hergestellt: $MgCl_2$ wurde in 95%igem Ethanol: Glycerin (4:1) gelöst, DNCB in 4:1 Aceton-Olivenöl. Danach wurden beide Lösungen entsprechend der in Tabelle 1 angegebenen Konzentrationen gemischt. DNCB allein wurde im Gemisch Ethanol : Glycerin : Aceton : Olivenöl aufgetragen. 24 Stunden später (Tag +6) wurde mit einem Mikrometer (Fa. Peacok Inc., Tokyo) die Ohrdicke gemessen (Post-DNCB-Meßwert). Die Ohrdickenzunahme resultierte als Folge der Kontaktsensibilisierung gegenüber DNCB infolge der primären dreitägigen DNCB-Applikation (Tag -3, -2, -1). Sie ergibt sich aus der Differenz des Post-DNCB-Meßwertes minus des Prä-DNCB-Meßwertes. Wie ersichtlich (Tabelle 1), hemmt $MgCl_2$ die durch DNCB ausgelöste entzündliche Reaktion entscheidend. Im Gegensatz dazu hat NaCl keine diesbezügliche Wirkung (Tabelle 1, Vergleich Nr. 7 mit Nr. 8 : p 0,01).

$MgCl_2$ wurde deshalb in einer Konzentration von 28% angewandt, weil im entzündungshemmenden Was-ser des Toten Meeres $MgCl_2$ in dieser Konzentration enthalten ist. Entsprechend der vorgestellten experimentellen Befunde dürfte es sich bei dem heilsamen Salz des Wassers des Toten Meeres um $MgCl_2$ handeln.

EP 0 439 640 B1

DNCB - Sensibilisierung und Hemmung der "Challenge" - Antwort durch MgCl₂ (BALB/c-Mäuse)

| Tiergruppe (1)* | | Ohrdickenzunahme (2)* |
| Nr. | "Challenge" Substanzen | (mm +/- SE) |
| --- | --- | --- |
| 1 | DNCB 0,5 % | 0,15 +/- 0,02 |
| 2 | DNCB 0,5 %  + MgCl₂ 28 % | 0,17 +/- 0,03 |
| 3 | DNCB 0,25% | 0,14 +/- 0,02 |
| 4 | DNCB 0,25%  + MgCl₂ 28 % | 0,07 +/- 0,02 |
| 5 | DNCB 0,125 % | 0,14 +/- 0,02 |
| 6 | DNCB 0,125 %  + MgCl 28 % | 0,02 +/- 0,01 |
| 7 | DNCB 0,125 %  + MgCl₂ 14 % | 0,06 +/- 0,02 |
| 8 | DNCB 0,125 %  + NaCl 14 % | 0,11 +/- 0,02 |
| 9 | Kontrolle (DNCB 0,25 %, keine primäre DNCB-Sensibilisierung) | 0,02 +/- 0,01 |

5

```
(1)* Jede Tiergruppe umfaßt 5 männliche BALB/c-Mäuse im Alter
     zwischen 4 und 6 Wochen.


(2)* Post-DNCB-Meßwert minus Prä-DNCB-Meßwert.
     Statistische Unterschiede (WILCOXON-Test): Nr. 1/2: keine
     signifikante Differenz; Nr. 3/4 : p < 0,05; Nr. 5/6 : p <
     0,001; Nr. 5/7 : p < 0,01; Nr. 7/8 p < 0,01.
```

Beispiel 2:

Epikutane Testung - Applikation von Formaldehyd in Kombination mit bzw. ohne $MgCl_2$bei Patienten mit Formaldehyd-Allergie

Als Folge der Applikation von Formaldehyd zusammen mit $MgCl_2$ bei Patienten mit einer Formaldehyd-Allergie resultiert eine deutliche, $MgCl_2$-konzentrationsabhängige verminderte Kontaktsensibilisierungs-Reaktion. Zwecks Nachweises der entzündungshemmenden Wirkung von $MgCl_2$ wurde $MgCl_2$ in einer End-konzentration von 28% bzw. 14% in die Salbengrundlage Unguentum emulsificans aquosum inkorporiert.

Die Zusammensetzung der Salbengrundlage ist wie folgt: Alcoholum emulsificans non ionogenicum 21,0 %, Cera perliquida 10,0 %, Glycerolum 5 %, Propylhydroxybenzionicum 0,06 %, Methylhydroxybenzionicum 0,14 %, Ethanolum (90 Vol%) 1,8 %, Aqua bidestillata 62,0 %. Formaldehyd wurde 5 %ig in die Salbengrundlage inkorporiert.

Applikation von Formaldehyd in Kombination mit bzw. ohne $MgCl_2$ bei einem Patienten mit Formaldehyd-Allergie

```
(1) Rp. Formaldehyd                        5,0
        MgCl₂                             28,0
        Ungt. emulsificans aquos.ad      100,0


(2) Rp. Formaldehyd                        5,0
        MgCl₂                             14,0
        Ungt. emulsificans aquos.ad      100,0


(3) Rp. Formaldehyd                        5,0
        Ungt. emulsificans aquos.ad      100,0
```

Aufgetragene Absolutmengen auf ein Hautreal von 0,5 x 0,5 cm : Formaldehyd : 0,45 mg; $MgCl_2$ x 6 $H_2O$ (28 %) : 2,52 mg = 1,38 Mol/l; $MgCl_2$ x 6 $H_2O$ (14 %) : 1,26 mg = 0,69 Mol/l.

Beispiel 3 :

Epikutane Testung - Applikation von Nickelsulfat in Kombination mit bzw. ohne $MgCl_2$bei Patienten mit Nickel-Allergie und Vergleich der entzündungshemmenden Wirkung von $MgCl_2$mit der des NaCl

Als Folge der Applikation von Nickelsulfat ($NiSO_4$ x 7 $H_2O$) zusammen mit $MgCl_2$ bei Patienten mit Nickel-Allergie resultierte ebenfalls eine gehemmte Kontaktsensibilierungsreaktion. Diese war abhängig von der $MgCl_2$ - Konzentration. Im Gegensatz zum $MgCl_2$ hemmte NaCl die Nickel-induzierte entzündliche Reaktion nicht.

6

Applikation von Nickelsulfat ($NiSO_4$ x 7 $H_2O$) in Kombination mit bzw. ohne $MgCl_2$ und Vergleich der entzündungshemmenden Wirkung von $MgCl_2$ mit der des NaCl. Salbengrundlage wie im Beispiel Nr. 1. Applizierte Absolutmengen auf ein Hautareal von 0,5 x 0,5 cm: Kontrolle: Applikation nur der Salbengrundlage

```
NiSO₄      5 % : 0,45 mg NiSO₄ x 7H₂O  (= 0,18 Mol/1)


MgCl₂     28 % : 2,52 mg MgCl₂ x 6H₂O  (= 1,38 Mol/1)
               + 0,45 mg NiSO₄ x 7H₂O  (= 0,18 Mol/1)


MgCl₂     14 % : 1,26 mg MgCl₂ x 6H₂O  (= 0,69 Mol/1)
               + 0,45 mg NiSO₄ x 7H₂O  (= 0,18 Mol/1)


NaCl      14% :  1,26 mg NaCl           (= 2,4  Mol/1)
               + 0,45 mg NiSO₄ x 7H₂O  (= 0,18 Mol/1)
```

Gemäß der Erfindung wird vorgeschlagen, Mg-Ionen in Form eines Salzes oder in seinen verschiedenen Salzen in wasserhaltige Salbengrundlagen, Lösungen, Zahncremes, Grundlagen für Körperwaschmittel oder Wasser einzuarbeiten.

In einer weiteren Lösung der Erfindung wird vorgeschlagen Mg-Ionen in Form eines Salzes oder in seinen verschiedenen Salzen in Kombination mit Ionen von Cd, Co und Mn bzw. deren Salze in wasserhaltige Salbengrundlagen, Lösungen, Zahncremes, Grundlagen für Körperwaschmittel oder Wasser einzuarbeiten.

Magnesium bzw. dessen Salze sind deshalb als entzündungshemmende Dermatika besonders geeignet, weil sie für den Menschen nicht toxisch sind.

Beispiel 4:

Herstellung entzündungshemmender Dermatika für die äußerliche Anwendung

```
Rp.   MgCl₂                                  28,0  (1)*
      Ungt. emulsificans aquos. ad          100,0


      (1)* Konzentrationsbereiche : 1,0 bis 28,0
           Gewichtsprozente


Rp.   MgCl₂                                  14.0  (2)*
      Ungt. Alcohol. Lanae aquos. ad        100,0


      (2)* Konzentrationsbereiche : 1,0 bis 14,0
           Gewichtsprozente


Rp.   MgCl₂                                  28,0  (1)*
      Aqua ad                               100,0


      (1)* Konzentrationsbereiche : 1,0 bis 28,0
           Gewichtsprozente
```

Anstelle von $MgCl_2$ können, wie oben ausgeführt, wegen der prinzipiell gleichartigen Wirkung auch folgende Ionen in Kombination mit Mg-$Cl_2$ äußerlich angewendet werden, nämlich Cd, Co, Mn.

$MgCl_2$ sollte das heilwirksame Salz des Wassers des Toten Meeres sein. Durch UV-Licht kann dessen entzündungshemmende Wirkung noch verstärkt werden, beispielsweise durch die Thalasso-Helio-Therapie, Sole-Photo-Therapie, Balneo-Photo-Therapie.

**Patentansprüche**

1. Verfahren zur äußerlichen Anwendung bei der Therapie entzündlicher Hautkrankheiten sowie zur Abschwächung allergischer entzündlicher Raktionen der Haut enthaltend aus 1 bis 30 Gew.-% Magnesiumchlorid aber keine Calciumsalze sowie Trägersubstanzen.

2. Mittel gemäß Anspruch 1, dadurch gekennzeichnet, daß es zusätlich Cadmium-, Kobalt- oder Manganlonen oder Gemische derselben enthält.

3. Mittel gemäß einem der Ansprüche 1 oder 2, dadurch gekennzeichnet, daß als Trägersubstanzen verwendet werden Salbengrundlagen, Lösungen, Wässer, Zahncremes, Grundlagen von Körperwaschmitteln oder Kosmetika.

4. Mittel gemäß einem der Ansprüch 1 bis 3, dadurch gekennzeichnet, daß es besteht aus Magnesiumchlorid in Ungentum emulsificans aquosum oder Ungentum Alcoholicum Lanae aquosum.

5. Verwendung von 1 bis 30 Gew.-% Magnesiumchlorid und Trägersubstanzen zur herstellung von Mitteln zur äußerlichen Auwendung bei der Therapie entzündlicher Hautkrankheiten sowie zur Abschwächung allergischer entzündlicher Reaktionen der Haut, wobei die mittel keine Calciumsalze enthalten.

EP 0 439 640 B1

## Claims

1. An agent for topical application in the therapy of inflammatory skin diseases and for mitigation of allergic inflammatory skin reactions, containing from 1 to 30% by weight of magnesium chloride, but no calcium salts, and vehicles.

2. The agent according to claim 1, characterized in that it additionally contains cadmium, cobalt or manganese ions or mixtures thereof.

3. The agent according to one of claims 1 or 2, characterized in that as the vehicles, there are used ointment bases, solutions, lotions, toothpastes, bases of body washing agents or cosmetics.

4. The agent according to one of claims 1 to 3, characterized in that it consists of magnesium chloride in hydrous emulsifying ointment or oily cream.

5. The use of from 1 to 30% by weight of magnesium chloride and vehicles for the preparation of agents for topical application in the therapy of inflammatory skin diseases and for mitigation of allergic inflammatory skin reactions, wherein said agents do not contain any calcium salts.

## Revendications

1. Remède destiné à être utilisé pour une application externe en thérapie de dermatites inflammatoires et pour atténuer des réactions inflammatoires allergiques de la peau, comprenant de 1 à 30 % en poids de chlorure de magnésium, mais pas de sels de calcium, et des substances vectrices.

2. Remède selon la revendication 1, caractérisé en ce qu'il comprend en outre des ions cadmium, cobalt ou manganèse, ou un mélange desdits ions.

3. Remède selon l'une des revendications 1 ou 2, caractérisé en ce que des bases de pommade, des solutions, des eaux, des dentifrices, des bases de produits de lavage corporel ou de produits de beauté sont utilisés comme substances vectrices.

4. Remède selon l'une des revendications 1 à 3, caractérisé en ce qu'il comprend du chlorure de magnésium dans un onguent consistant en un émulsifiant aqueux ou en alcool de laine aqueux.

5. Utilisation de 1 à 30 % en poids de chlorure de magnésium et de substances vectrices pour fabriquer des remèdes destinés à être utilisés pour une application externe en thérapie de dermatites inflammatoires ainsi que pour atténuer des réactions inflammatoires allergiques de la peau, le remède ne contenant alors pas de sels de calcium.

9